Europäisches Patentamt

European Patent Office

Office européen des brevets

(19)

(11) Numéro de publication: **0 162 763**
**B1**

(12) **FASCICULE DE BREVET EUROPEEN**

(45) Date de publication du fascicule du brevet: **26.10.88**

(21) Numéro de dépôt: **85400827.3**

(22) Date de dépôt: **26.04.85**

(51) Int. Cl.⁴: **A 61 F 5/04,** A 61 F 5/01, B 32 B 5/18

(54) **Matériau pour la confection d'articles de maintien de la tête ou de membres de l'homme ou d'animaux et articles confectionnés à partir de ce matériau.**

(30) Priorité: **07.05.84 FR 8407031**

(43) Date de publication de la demande:
**27.11.85 Bulletin 85/48**

(45) Mention de la délivrance du brevet:
**26.10.88 Bulletin 88/43**

(84) Etats contractants désignés:
**BE CH DE IT LI**

(56) Documents cités:
**EP-A-0 005 615**
**FR-A-1 515 909**
**FR-A-2 501 501**
**FR-A-2 507 887**
**GB-A-2 108 849**

(73) Titulaire: **ETABLISSEMENTS THUASNE & CIE**
**27, rue de la Jomayère**
**F-42031 St. Etienne Cedex (FR)**
(73) Titulaire: **THUASNE-PARIS**
**73, rue du Faubourg St. Martin**
**F-75010 Paris (FR)**

(72) Inventeur: **Picolet, Jean-Pierre**
**3 Rue des Glycines**
**F-42270 Saint Priest-en-Jarez (FR)**
Inventeur: **Rudloff, Jean-Loup**
**29, rue de Stalingrad**
**F-92000 Nanterre (FR)**
Inventeur: **Courtet, François**
**La Cote**
**F-43330 St-Bonnet-Les-Oules (FR)**

(74) Mandataire: **Coutel, Jean-Claude**
**Cabinet AYMARD & COUTEL 20, rue Vignon**
**F-75009 Paris (FR)**

Courier Press, Leamington Spa, England.

## Description

L'invention est relative à un matérieau pour la confection d'articles de maintien de la tête ou de membres de l'homme ou d'animaux. Elle s'applique également aux articles confectionnés à partir de ce matériau.

Parmi ces matériaux, l'invention concerne ceux qui sont du type stratifié décrit dans le document FR—A—2 501 501.

Le matériau stratifié selon cette demande de brevet citée est constitué par un panneau stratifié formé par l'assemblage notamment d'une couche extérieur de matière plastique sous forme de mousse dure, d'une feuille de métal déformable mais non élastique, et d'une couche intérieure de matière plastique sous forme de mousse souple.

A l'usage, il a été démontre que les articles confectionnés du type général "gouttières" avaient une utilisation possible en milieu hospitalier, ayant alors un rôle de maintien des positions physiologiques sur les personnes privées de leur connaissance (usage post-opératoire) ou lors de ré-éducations fonctionnelles (maintien de posture).

Or de telles utilisations ne demandent pas un modelage important, par contre une certaine élasticité est souhaitée pour donner à l'article une action dynamique. Il est nécessaire également, pour augmenter l'utilisation de l'article, de trouver un matériau qui ne présente pas de rupture après un nombre élevé de pliages au même endroit.

Pour ces raisons l'invention a pour object de substituer, totalement ou partiellement, à la feuille de métal du stratifié de la demande de brevet ci-dessus une feuille de matière plastique, en polyéthylène ou en polypropylène ou autre matière plastique élasticité équivalente.

On peut choisir, notamment, une feuille de polyéthylène, d'épaisseur voisine de 2 mm mais pouvant varier suivant l'action dynamique souhaitée. Cette feuille viendre en association, ou en remplacement, de la feuille métallique et, comme cette dernière, se présentera sous forme prédéterminée qui correspond à la conformation de l'article à confectionner.

D'autres améliorations ont également été apportées en fonction de l'usage particulier d'un article.

Les caractéristiques et avantages de l'invention seront bien comprises grâce à la lecture de la description, qui va suivre, et à l'examen des dessins annexés, concernant deux exemple nullement limitatifs d'articles confectionnés.

Il s'agit, dans le premier exemple, d'un collier cervical et, dans le seconde, d'une gouttière constituant une "botte orthopédique".

Sur les dessins:

Fig. 1 est une vue en plan, partiellement arrachée, d'un collier à plat, permettant de voir la découpe particulière en T de la fueille de polyéthylène;

Fig. 2 montre la coupe de ce même collier suivant II—II de la Fig. 1, et met en évidence les améliorations apportées par rapport au stratifié décrit dans la demande de brevet ci-dessus.

Fig. 3 montre le colliere mis en place;

Fig. 4 est une vue écorchée d'une gouttière du type "botte orthopédique";

Fig. 5 est une vue en coupe suivant V—V de la Fig. 4, mettant en évidence les différents éléments constituant le stratifiè;

Fig. 6 montre un exemple de mise en place de la gouttière sur un patient, permettant un exercice dynamique de l'articulation; et

Fig. 7 est une vue en coupe partielle montrant le rôle des différents éléments, la gouttière étant en place.

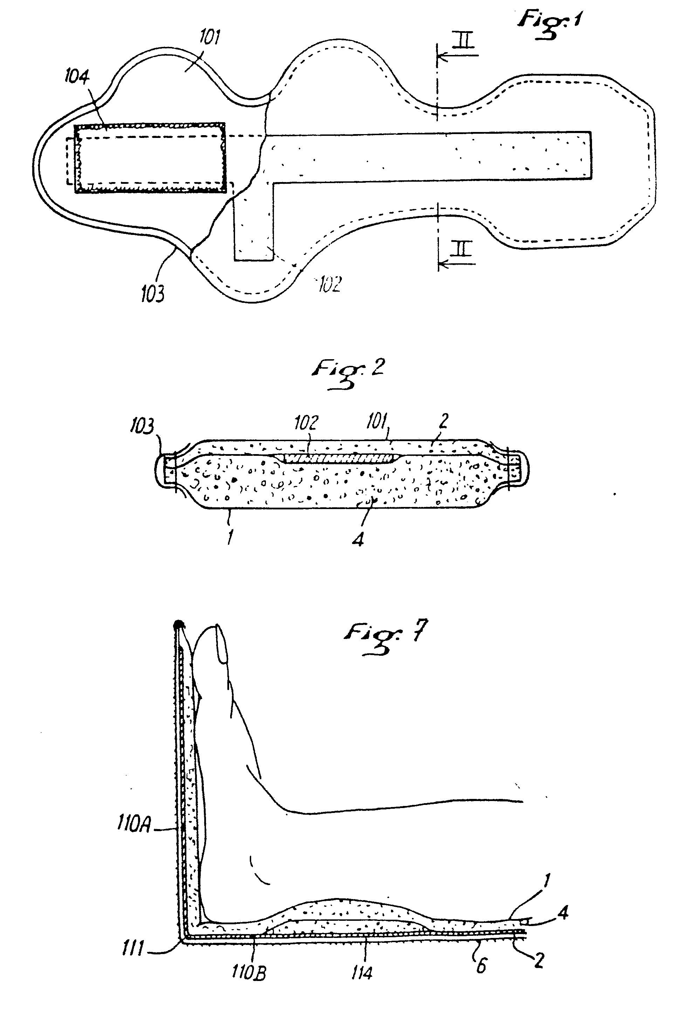

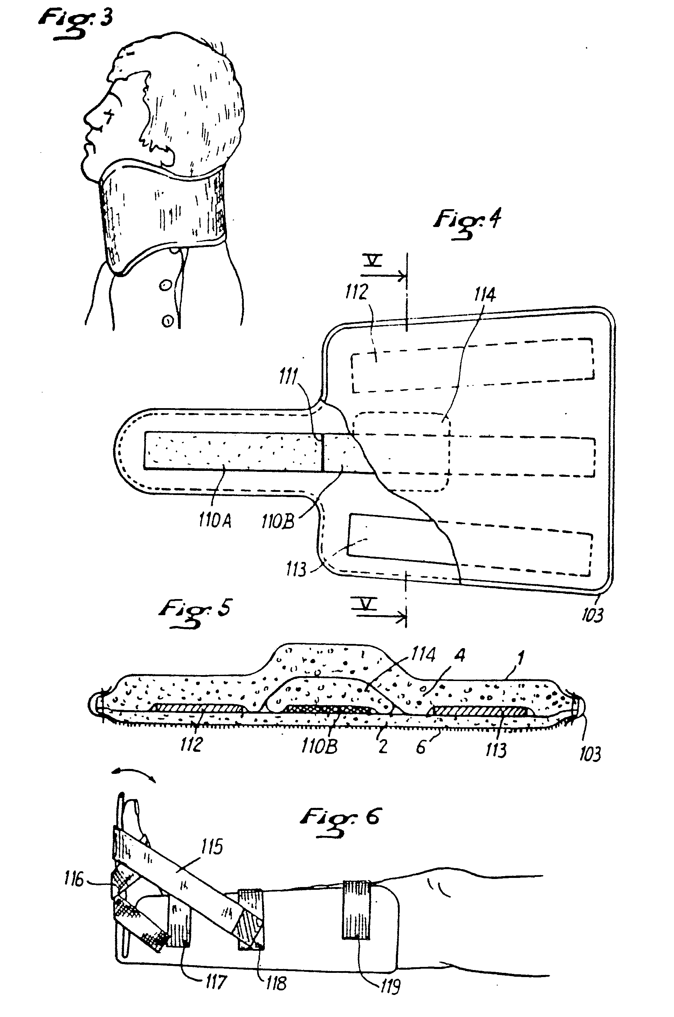

### Exemple 1
### Collier cervical (Figs. 1—3)

On remarque ainsi l'utilisation de la feuille de polyéthylène 102, à la place de la feuille méallique prévue dans la demande de brevet ci-dessus. Le collier s'adapte ainsi facilement à la forme du cou et retrouve une forme plane lorsqu'on l'enlève.

Les plaies à la tête saignant beaucoup, une adaptation du stratifié a été faite pour permettre un nettoyage facile de l'article. La couche de mousse de matière plastique dure 2 a été recouverte non par du tricot prévu dans la demande de brevet ci-dessus, mais par une feuille souple et lisse 101, en tissu enduit par du polychlorure de vinyle. Enfin, un bordage cousu 103 de l'article également en tissu enduit de polychlore de vinyle évit la salissure latérale du stratifié.

Cette nouvelle surface, enduite de polychlorure de vinyle ne permettant plus l'accrochage des brides, une surface auto-agrippante 104 du type mis dans le commerce sous l'appellation "Velcro" a été rapportée au strict endroit nécessaire.

### Exemple 2
### Gouttière du type "botte orthopédique"
### (Figs. 4 à 7)

C'est un exemple d'association d'une feuille métallique 110 A et 110 B et d'une feuille de polyéthylène de 2 mm sous forme de deux bandes 112 et 113. On obtient ainsi une bonne rigidité de la tige de la botte et un maintien plastique latéral. On remarquera la jonction entre les parties 110 A et 110 B faite par une charnière 111, cette dernière permettant l'utilisation de la botte pour un exercise dynamique de l'articulation. Un contrôle de la posture est exercé par l'axe de la charnière et par les volets latéraux de la gouttière.

Une limitation de l'amplitude du mouvement peut être fixée par le médecin grâce à la bride 116 et enfin, un rappel du pied est assuré par une bride élastique 115. La force de cette dernière dépendra de l'exercice souhaité; on pourra également jouer sur son allongement. Si l'effet dynamique n'est pas recherché, la bride 115 peut être en tissu rigide. Des brides pouvant être appelées à se recouvrir, il a été utilisé du ruban "Velcro double face" pour la réalisation des

brides 117, 118 et 119. On garde ainsi la possibilité de fixer les brides de réglage 115 et 116 en tous points de la botte.

La dernière modification porte sur l'incorporation, dans le stratifié, d'un coussinet 114, réalisé dans le même matériau de confort que la mousse 4 de la demande de brevet ci-dessus. Son rôle est double: en premier lieu, il permet à l'article de venir épouser plus fidèlement la forme anatomique de la partie du corps à lquelle il est destiné; on réalise ainsi une meilleure répartition du poids de la jambe au contact de la botte et il contribue ainsi à éviter l'escarre du talon qui aurait lieu en cas de contact ponctuel. Il joue également un rôle de blocage du talon et évite ainsi un déplacement de la botte lors d'exercices dynamiques.

D'autres modifications ont été envisagées, notamment pour la lutte contre les escarres. Outre l'adjonction de capitonnage décrit dans l'exemple 2, il a été réalisé, sur d'autres articles, diverses modifications ayant la même finalité, à savoir un évidement dans le matériau en regard de la zone menacée, et l'utilisation d'une doublure du matériau en contact avec la peau avec un matériau approprié tel que feuille de mousse de latex ou tissu "peau de mouton", ou tout autre matériau permettant de lutter contre les escarres.

On peut, en outre, envisager l'adjonction d'accessoires de fixation pour utilisation spécifique (par exemple utilisation conjointe avec fixateur externe).

## Revendications

1. Matériau pour la confection d'articles de maintien de la tête ou de membres de l'homme ou des animaux, ce matérieau étant constitué par l'assemblage d'une couche extérieure (2) de matière plastique sous forme de mousse dure, d'une feuille intermédiaire (102, 112, 113) et d'une couche intérieure (4) sous forme de mousse souple, caractérisé par le fait que la feuille intermédiaire (102, 112, 113) est composée de matière plastique en polyéthylène ou polypropylene ou autre matière plastique d'élasticité équivalente.

2. Matériau selon la revendication 1, caractérisé par le fait que le face distale de la couche extérieure (2) est recouverte d'une feuille souple et lisse (101), tel qu'un tissu enduite de polychlorure de vinyle, un matériau d'accrochage des brides étant rapporté aux seuls endroits nécessaires.

3. Matériau selon l'une des revendications 1 et 2, caractérisé en ce qu'un coussinet (114) supplémentaire est incorporé pour que l'article épouse ainsi au mieux les formes anatomiques dans le but de diminuer le risque d'escarres et d'empêcher le déplacement de l'article lors de son utilisation.

4. Matériau selon l'une des revendications 1 et 2, caractérisé en ce que la feuille intermédiaire, incorporée dans le stratifié, comporte une articulation par charnière (111) permettant une utilisation dynamique de l'article sans risque de rupture de la feuille.

5. Matérieau selon l'une des revendications 1 et 2, caractérisé en ce que la prévention des escarres est obtenue par la présence d'un évidement dans le matériau ou par l'utilisation d'une doublure de la partie en contact avec la peau.

6. Matérieau selon l'une des revendications 1 à 5, caractérisé par le fait qu'il comporte également, en association avec les autres couches, une couche métallique (110 A, 110 B).

7. Articles, notamment gouttières, attelles et éléments de posture confectionnés à partir d'un matériau selon l'une quelconque des revendications précédentes.

## Patentansprüche

1. Material zur Herstellung von Gegenständen zum Halten des Kopfes oder von Gliedern des Menschen oder von Tieren, wobei dieses Material durch die Verbindung einer äußeren Schicht (2) aus Kunststoffmaterial in Form von Hartschaum, einer Zwischenfolie (102, 112, 103) und einer inneren Schicht (4) in Form einer weichen Masse gebildet ist, dadurch gekennzeichnet, daß die Zwischenfolie (102, 112, 113) aus Kunststoffmaterial von Polyethylen oder Polypropylen oder aus einem anderen Kunststoffmaterial mit gleichwertiger Elastizität zusammengesetzt ist.

2. Material nach Anspruch 1, dadurch gekennzeichnet, daß die distale Oberfläche der äußeren Schicht (2) mit einer weichen und glatten Folie (101), wie etwa einem mit Polyvinylchlorid überzogenen Stoff, bedeckt ist, wobei ein Material zur Befestigung von Binden lediglich an den dafür notwendigen Stellen angebracht ist.

3. Material nach einem der Ansprüche 1 und 2, dadurch gekennzeichnet, daß ein zusätzliches Polster (114) eingearbeitet ist, damit der Gegenstand sich um so besser an die anatomischen Formen anschließt zu dem Zweck, das Risiko von Abschürfungen zu vermindern und die Verschiebung des Gegenstandes beim Gebrauch zu verhindern.

4. Material nach einem der Ansprüche 1 und 2, dadurch gekennzeichnet, daß die in den Schichtstoff eingearbeitete Zwischenfolie ein Scharniergelenkverbindung (111) aufweist, welche einen dynamischen Gebrauch des Gegenstandes ohne Risiko eines Reißens der Folie gestattet.

5. Material nach einem der Ansprüche 1 und 2, dadurch gekennzeichnet, daß die Verhinderung von Abschürfungen durch die Anwesenheit einer Ausnehmung in dem Material oder durch die Verwendung eines Futters an dem mit der Haut in Kontakt stehenden Abschnitt erreicht wird.

6. Material nach einem der Ansprüche 1 bis 5, dadurch gekennzeichnet, daß es zusammen mit den anderen Schichten auch eine metallische Schicht (110A, 110B) aufweist.

7. Gegenstände, insbesondre Schienen, Stützen and Halteelemente, welche aus einem Material nach einem der vorhergehenden Ansprüche hergestellt sind.

## Claims

1. Material for the manufacture of articles for supporting the heads or members of persons or animals, this material being constituted by the assembly of an outer layer (2) of plastic material in the form of hard foam, of an intermediate sheet (102, 112, 113) and of an inner layer (4) in the form of a flexible foam, characterized by the fact that the intermediate sheet (102, 112, 113) is composed of a plastic material in polyethylene or polypropylene or in any plastic material of equivalent elasticity.

2. Material according to claim 1, characterized in that the distal face of the outer layer (2) is covered with a flexible and smooth sheet (101) such as a fabric covered with vinyl polychloride, a material for fastening the straps being attached only at the necessary locations.

3. Material according to any one of claims 1 and 2, characterized in that an additional pad (114) is incorporated so that the article thus matches as best as possible the anatomic shapes with a view to reducing the danger of scabs and preventing the displacement of the article when being used.

4. Material according to one of claims 1 and 2, characterized in that the intermediate sheet, incorporated in the layered material, includes a hinge articulation (111) allowing a dynamic use of the article without danger of rupture of the sheet.

5. Material according to one of claims 1 and 2, characterized in that the prevention of scabs is obtained by the presence of a recess in the material or by using a lining of the part in contact with the skin.

6. Material according to one of claims 1 to 5, characterized by the fact that it includes also, in association with the other layers, a metallic layer (110A, 110B).

7. Articles, notably cradles, splints and posture elements made from a material according to any one of the preceding claims.

*Fig. 1*

*Fig. 2*

*Fig. 7*

0 162 763

*Fig. 3*

*Fig. 4*

V
112        114

111

110A   110B

113

103

*Fig. 5*

114  4   1

112        110B   2   6    113        103

*Fig. 6*

115
116

117     118        119

2